# EUROPEAN PATENT SPECIFICATION

(11) **EP 4 321 193 B1**
(45) Date of publication and mention of the grant of the patent: **24.09.2025**
(21) Application number: 23765128.6
(22) Date of filing: 31.05.2023
(51) Int. Cl.: A61M 15/00, A61M 11/02, A61M 15/06, A61M 16/20

(54) **INHALER**
INHALATOR
INHALATEUR

(30) Priority: 01.07.2022 KR 20220081055
(43) Date of publication of application: 14.02.2024
(73) Proprietor: KT & G Corporation, Daejeon 34337 (KR)
(72) Inventor: KIM, Tae Heon, Yuseong-gu, Daejeon 34128 (KR); KIM, Jae Hyun, Yuseong-gu, Daejeon 34128 (KR); LEE, Mi Jeong, Yuseong-gu, Daejeon 34128 (KR); JEONG, Minseok, Yuseong-gu, Daejeon 34128 (KR); JUNG, Yongmi, Yuseong-gu, Daejeon 34128 (KR); JEOUNG, Eunmi, Yuseong-gu, Daejeon 34128 (KR); CHUNG, Tae Young, Yuseong-gu, Daejeon 34128 (KR); HAN, Seung Kyu, Yuseong-gu, Daejeon 34128 (KR)
(74) Representative: Ter Meer Steinmeister & Partner
(86) International application number: PCT/KR2023/007445
(87) International publication number: WO 2024/005384

(56) References cited:
- JP-A- 2014 532 445
- JP-A- 2020 151 022
- KR-A- 20090 094 111
- KR-B1- 100 399 845
- US-A1- 2003 183 225
- US-A1- 2007 074 718

## Description

### Technical Field

Disclosed is an inhaler.

### Background Art

In general, an inhaler is a device used to inhale a composition such as medication through the oral cavity or nasal cavity as a liquid or gas in the process of inhalation. Such an inhaler may include a container accommodating an inhalable composition, and the composition may be sprayed from the container through a thin tube to the oral cavity or nasal cavity through an intake to be inhaled by a user.

The above description has been possessed or acquired by the inventor(s) in the course of conceiving the present disclosure and is not necessarily an art publicly known before the present application is filed.

Prior Document: Korean Patent Gazette No. 10-2021229 (Published on September 11, 2019)
US 2007/074718 A1 relates to a medication inhaler for delivery of a medication to a patient from a canister having a valve stem, such as a MDI canister. The medication inhaler includes a chamber body having a fresh air inlet and a hollow spacer chamber. The inhaler further includes a canister retainer configured to move the canister between operational and stored positions, and an actuation lever for actuation of the valve stem to deliver a medication fluid into the hollow spacer chamber. The hollow spacer chamber tapers. In addition or alternatively, the chamber body includes a nozzle having an outlet, and a fresh air inlet having an outlet proximate the outlet of the nozzle. In addition or alternatively, the medication inhaler includes a canister adapter for accommodation of various size canisters. In addition or alternatively, the chamber body includes an inlet portion having a geometry for guiding the valve stem.

### Disclosure of the Invention

### Technical Goals

An object according to an embodiment is to provide an inhaler capable of performing smooth filling by discharging a residual gas in a reservoir before filling the reservoir with an inhalable composition.

The technical tasks obtainable from the present disclosure are non-limited by the above-mentioned technical tasks. And, other unmentioned technical tasks can be clearly understood from the following description by those having ordinary skill in the technical field to which the present disclosure pertains.

### Technical Solutions

An inhaler according to an embodiment for achieving the above objects includes: a housing having one surface, the other surface opposite to the one surface, and a plurality of side surfaces connecting the one surface and the other surface; a reservoir which is disposed inside the housing and stores an inhalable composition; a canister which accommodates the inhalable composition to be stored in the reservoir and is connected to the reservoir to be opened or closed; a relief valve which is disposed to be spaced apart from the canister and provided to open and close the reservoir to the outside; and a lever which controls an opening and closing operation of at least one of the canister or the relief valve. The lever controls the canister to be opened after the relief valve is opened.

According to an aspect, the inhaler may further include a relief bar having one end coupled to the relief valve and extending toward the lever. The relief bar may be positioned between the lever and the canister.

According to an aspect, the lever may further include a support member which extends in a first direction toward the canister; and a protrusion member having an end portion rotatably coupled to the support member around a rotation shaft provided in the support member, and the other end of the relief bar may be positioned between the protrusion member and the other surface of the housing.

According to an aspect, when the lever is pressed, the protrusion member may move the relief bar in a second direction toward the one surface of the housing while being moved in the first direction.

According to an aspect, when the lever is pressed in the first direction, the support member may restrict rotation of the protrusion member, and when the lever returns to an original position, the support member may allow the rotation of the protrusion member.

According to an aspect, the protrusion member may be disposed so that rotation in a direction toward the canister is not restrained and rotation in a direction away from the canister is restrained in a state where the end portion stands toward the one surface of the housing.

According to an aspect, when the canister is opened, the protrusion member may be positioned between the relief bar and the canister.

According to an aspect, the lever may further include a wedge member extending toward a bottom surface of the canister, and a distance between the wedge member and the bottom surface of the canister may be greater than a distance between the protrusion member and the relief bar.

According to an aspect, the wedge member may include an inclined surface inclined to be adjacent to the bottom surface along the first direction, and when the lever is further pressed in the first direction in a state where the inclined surface comes into contact with the bottom surface, the inclined surface may move the canister in the second direction toward the one surface of the housing.

According to an aspect, when the lever reaches a first position in which the relief valve is opened, the inhalable composition remaining in the reservoir may be discharged to the outside, and when the lever reaches a second position in which one side of the canister is opened, the reservoir may be filled with the inhalable composition accommodated in the canister.

According to an aspect, the relief bar may open the relief valve when the relief bar moves in a second direction toward the one surface of the housing, and close the relief valve when the relief bar returns to an original position.

According to an aspect, the canister may include: an accommodation portion which accommodates the inhalable composition; and an injection hole having one side connected to the reservoir and the other side positioned in the accommodation portion, and the injection hole may be opened or closed to allow or restrict movement of the inhalable composition to the reservoir.

According to an aspect, the injection hole may be opened when the accommodation portion moves in a second direction from the other surface to the one surface of the housing, and may be closed when the accommodation potion returns to an original position.

### Effects

According to the inhaler according to an embodiment, there is an effect of providing smooth filling by discharging the residual gas in the reservoir before filling the reservoir with the inhalable composition.

The effects of the inhaler are not limited to the above-mentioned effects, and other unmentioned effects can be clearly understood from the following description by one of ordinary skill in the art to which the present disclosure pertains.

### Brief Description of Drawings

FIG. 1 is a perspective view of an inhaler according to an embodiment.
FIG. 2 is a cross-sectional view of an inhaler according to an embodiment.
FIG. 3 illustrates a relief valve that operates in association with a lever.
FIG. 4 illustrates a two-step operation process of a lever.
FIG. 5 illustrates an opening or closing operation of a relief valve and a canister when a lever operates in the same order as in FIG. 4.

The accompanying drawings illustrate desired embodiments of the present disclosure.

### Best Mode for Carrying Out the Invention

Hereinafter, embodiments will be described in detail with reference to the illustrative drawings. Regarding the reference numerals assigned to the components in the drawings, it should be noted that the same components will be designated by the same reference numerals, wherever possible, even though they are shown in different drawings. Further, in the following description of the present embodiments, a detailed description of publicly known configurations or functions incorporated herein will be omitted when it is determined that the detailed description obscures the subject matters of the present embodiments.

In addition, the terms first, second, A, B, (a), and (b) may be used to describe constituent elements of the embodiments. These terms are used only for the purpose of discriminating one constituent element from another constituent element, and the nature, the sequences, or the orders of the constituent elements are not limited by the terms. When one constituent element is described as being "connected", "coupled", or "attached" to another constituent element, it should be understood that one constituent element can be connected or attached directly to another constituent element, and an intervening constituent element can also be "connected", "coupled", or "attached" to the constituent elements.

The same name may be used to describe an element included in the embodiments described above and an element having a common function. Unless otherwise mentioned, the descriptions on the embodiments may be applicable to the following embodiments and thus, duplicated descriptions will be omitted for conciseness.

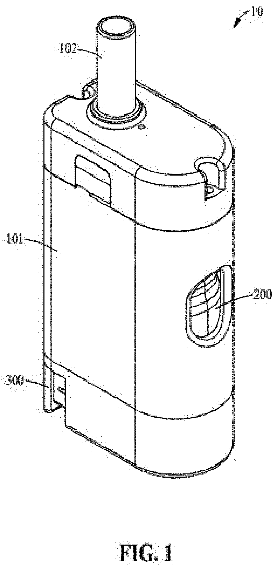

FIG. 1 is a perspective view of an inhaler 10 according to an embodiment.

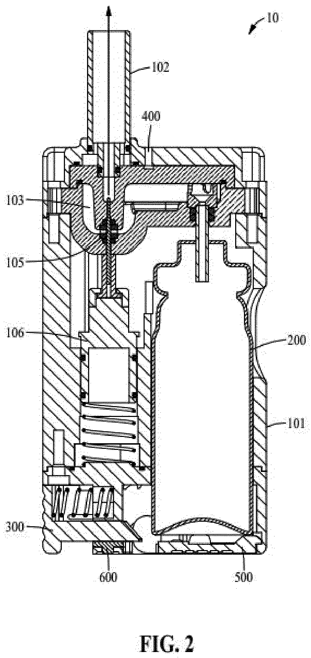

FIG. 2 is a cross-sectional view of the inhaler 10 according to an embodiment.

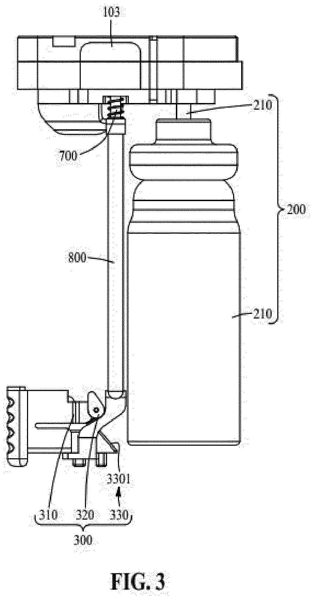

FIG. 3 illustrates a relief valve 700 that operates in association with a lever 300.

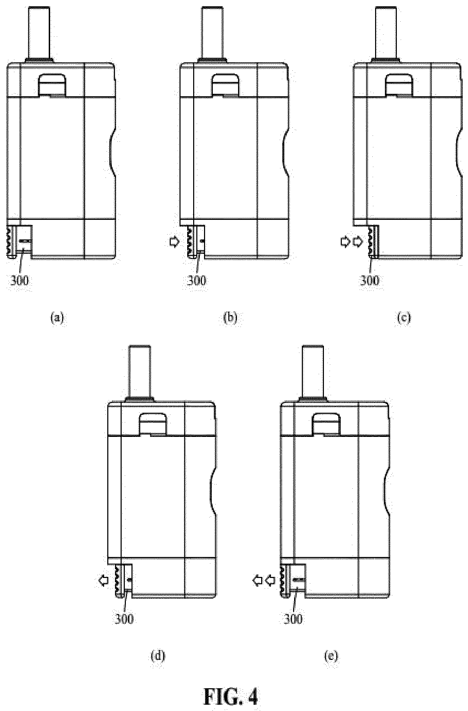

FIG. 4 illustrates a two-step operation process of the lever 300.

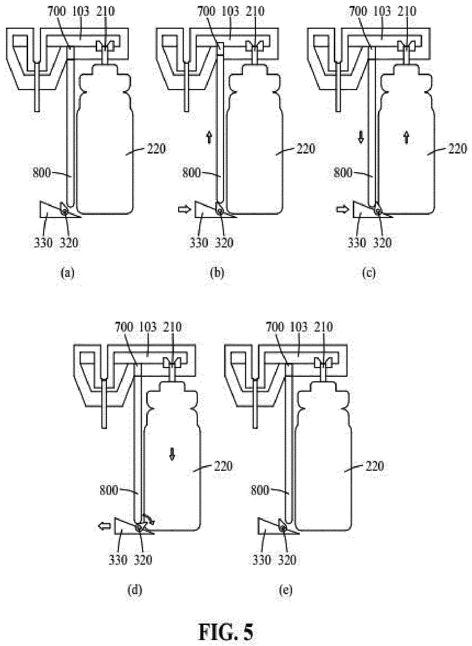

FIG. 5 sequentially illustrates an opening or closing operation of the relief valve 700 and a canister 200 when the lever 300 operates in the same order as in FIG. 4.

Referring to FIG. 1, the inhaler 10 according to an embodiment may include a housing 101, a mouthpiece 102, a reservoir 103, the canister 200, and the lever 300.

The housing 101 may include a first surface formed on one surface, a second surface opposite to the first surface, and a plurality of side surfaces connecting the first surface and the second surface. The first surface of the housing 101 may be, for example, a surface located on the top of the housing 101, and the second surface may be, for example, a bottom surface of the housing 101.

The mouthpiece 102 may be disposed on the first surface of the housing 101. A user may inhale an inhalable composition accommodated in the inhaler 10 through the mouthpiece 102. At this time, the user may inhale the composition, for example, in the form of an aerosol or in the form of a powder. Hereinafter, the inhaler 10 according to an embodiment will be described using the inhaler 10 that sprays an inhalable composition in the form of an aerosol as an example.

Referring to FIGS. 1 and 2, a reservoir 103 may be disposed inside the housing 101. Since one side of the canister 200 is connected to the reservoir 103, the inhalable composition accommodated in the canister 200 may be moved to and stored in the reservoir 103. In addition, as the user applies a suction force through the mouthpiece 102, the inhalable composition stored in the reservoir 103 may be discharged through the mouthpiece 102 in the form of an aerosol, and accordingly, the inhalable composition stored in the reservoir 103 may be gradually consumed.

The canister 200 may be mounted inside the housing 101. In this case, the canister 200 may be mounted interchangeably inside the housing 101. In addition, the canister 200 may accommodate an inhalable composition therein. A certain amount of the composition of the canister 200 may be filled in the reservoir 103.

The lever 300 may be pressed by the user to fill the inside of the reservoir 103 with the composition accommodated in the canister 200. The lever 300 may form one side surface of the housing 101 and may be positioned adjacent to the second surface. When the user presses the lever 300, the lever 300 may push up a bottom surface of the canister 200 so that an injection hole 220 of the canister 200 communicates with the reservoir 103, and the composition may move from the canister 200 to the reservoir 103 through the injection hole 220.

Hereinafter, a direction from one side surface of the housing 101 where the lever 300 is located to the canister 200 is defined as a first direction, and a direction from the second surface of the housing 101 to the first surface is defined as a second direction.

Meanwhile, the relief valve 700 may be provided on the reservoir 103, and a relief vent hole 400 may be provided on the first surface of the housing 101. The relief valve 700 may be interlocked with the lever 300 through a relief bar 800, a relief bar movement protrusion 320, or the like, and the relief valve 700 may operate to open before the lever 300 pushes the canister 200 up. Accordingly, a residual gas in the reservoir 103 may be discharged before the reservoir 103 is filled with the inhalable composition.

The relief valve 700 will be described in more detail with reference to FIGS. 3 to 5 below.

Referring to FIG. 2, in the inhaler 10 according to an embodiment, the user may visually identify a remaining amount of the composition stored in the reservoir 103 through a viewing window (not shown) provided on the housing 101. In addition, the inhaler 10 according to an embodiment may include the counter (not shown) that counts the number of times of filling in connection with a vertical movement of the canister 200 during the filling of the reservoir 103, and a display window (not shown) that displays a remaining amount of the composition in the canister 200. The composition stored in the reservoir 103 may be sprayed in the form of an aerosol as the user applies a suction force to the mouthpiece 102. At this time, an inhalation interlocking valve 105 that opens and closes the reservoir 103 by the suction force may operate, and the opening and closing operation of the inhalation interlocking valve 105 may be controlled by a piston 106. The inhaler 10 according to an embodiment may further include a cover 500 and a locking device 600 for preventing the canister 200 from being separated.

As described above, the inhaler 10 according to an embodiment uses a method of filling the reservoir 103 with the inhalable composition accommodated in the canister 200, and thus, if a residual gas remains in the reservoir 103 during repeated refilling, it may be not easy to fill the reservoir 103 with the composition of the canister 200. Accordingly, the relief valve 700 interlocked with the filling lever 300 may be applied to the inhaler 10 according to an embodiment to facilitate the filling of the reservoir 103.

Referring to FIG. 3, the relief valve 700 of the inhaler 10 according to an embodiment may be provided on the reservoir 103 to be spaced apart from the one side of the canister 200. The relief valve 700 may be opened or closed to allow the reservoir 103 to communicate with the outside or block the reservoir 103.

The opening or closing operation of the relief valve 700 may be controlled by the lever 300 described above. For example, when the lever 300 is pressed by the user, the lever 300 may control the relief valve 700 so that the relief valve 700 is opened first and then the canister 200 is opened.

As described above, as the relief valve 700 interlocked with the lever 300 is applied to the inhaler 10 according to an embodiment, the residual gas in the reservoir 103 may be discharged and the reservoir 103 may be smoothly filled with the inhalable composition from the canister 200.

The lever 300 may be pressed by the user. At this time, the lever 300 may move toward the canister 200 to reach a first position, and when the lever 300 is further pressed by the user, the lever 300 may reach the second position. When the lever 300 is in the first position, the relief valve 700 may be opened, and when the lever 300 is in the second position, the relief valve 700 may be closed and the injection hole 220 of the canister 200 may be opened.

Specifically, the relief valve 700 may be interlocked with the lever 300 through the relief bar 800 and may be opened or closed by raising or lowering of the relief bar 800. The relief bar 800 may be disposed between the lever 300 and the canister 200. The relief bar 800 may have one end coupled to the relief valve 700 and the other end extending toward the lever 300. The relief bar 800 may open the relief valve 700 as the relief bar 800 moves in the second direction, and may close the relief valve 700 as the relief bar 800 returns to its original position.

The lever 300 may include a support member 310 and a protrusion member 320.

The support member 310 may extend toward the canister 200.

The protrusion member 320 may be rotatably coupled to the support member 310 around a rotation shaft provided in the support member 310. The protrusion member 320 may have an end portion protruding in a radial direction from the rotation shaft at one side. The end portion of the protrusion member 320 may be disposed in a standing state toward the first surface of the housing 101.

The other end of the relief bar 800 may be positioned between the protrusion member 320 and the second surface of the housing 101. In addition, the end portion of the protrusion member 320 may be inclined in a direction away from the canister 200 with respect to the rotation shaft.

When the lever 300 is pressed in the first direction, the support member 310 may restrain the rotation of the protrusion member 320. That is, the rotation of the protrusion member 320 in a direction away from the canister 200 may be restrained in a state where the end portion stands toward the first surface of the housing 101.

Therefore, when the lever 300 is pressed, the protrusion member 320 also moves in the first direction to reach the first position, and at this time, the protrusion member 320 may come into contact with the relief bar 800 and push the relief bar 800 up in the second direction without rotating.

As a result, when the lever 300 reaches the first position, the inhalable composition remaining in the reservoir 103 may be discharged to the outside.

Meanwhile, when the lever 300 returns to its original position, the support member 310 may allow the rotation of the protrusion member 320. That is, the rotation of the protrusion member 320 in the direction toward the canister 200 may not be restrained in a state where the end portion stands up toward the first surface of the housing 101.

Accordingly, when the lever 300 moves from the second position or a position beyond the first position in a direction opposite to the first direction, the protrusion member 320 may pass through the relief bar 800 and return to its original position without pushing the relief bar 800 up.

Referring back to FIG. 3, the lever 300 may further include a wedge member 330 extending toward the bottom surface of the canister 200.

The wedge member 330 may include an inclined surface 3301 inclined to be more adjacent to the bottom surface of the canister 200 along the first direction.

Accordingly, when the lever 300 is further pressed in the first direction while the inclined surface 3301 is in contact with the bottom surface, the inclined surface 3301 may move the canister 200 in the second direction.

A distance between the inclined surface 3301 of the wedge member 330 and the bottom surface of the canister 200 may be greater than a distance between the protrusion member 320 and the relief bar 800. Accordingly, when the lever 300 is moved in the first direction, the protrusion member 320 may first come into contact with the other end of the relief bar 800 before the inclined surface 3301 comes into contact with the bottom surface of the canister 200. That is, the relief valve 700 provided on the reservoir 103 may be opened first, immediately before the lever 300 pushes the canister 200 to fill the reservoir 103.

Meanwhile, when the canister 200 is opened, that is, when the lever 300 reaches the second position and the inclined surface 3301 pushes the canister 200 up in the second direction, the protrusion member 320 may be positioned between the relief bar 800 and the canister 200.

Eventually, when the lever 300 reaches the second position, the reservoir 103 may be filled with the inhalable composition accommodated in the canister 200.

Referring back to FIG. 3, the canister 200 of the inhaler 10 according to an embodiment may include an accommodation portion 210 and the injection hole 220.

The accommodation portion 210 may accommodate an inhalable composition.

One side of the injection hole 220 may be connected to the reservoir 103 and the other side thereof may be positioned in the accommodation portion 210. The injection hole 220 may operate to be opened or closed with respect to the reservoir 103. For example, when the lever 300 is in the second position, the injection hole 220 may be opened. When the injection hole 220 is opened, the inhalable composition accommodated in the accommodation portion 210 may be allowed to move to the reservoir 103. On the other hand, when the lever 300 is not in the second position, the injection hole 220 may be closed. When the injection hole 220 is closed, a moving path of the inhalable composition to the reservoir 103 may be blocked, and accordingly, the filling of the reservoir 103 may be stopped.

The operation of opening and closing the injection hole 220 may be controlled according to whether the user presses the lever 300.

As described above, when the lever 300 is pressed, the inclined surface 3301 of the wedge member 330 may push up the bottom surface of the canister 200, that is, a bottom surface of the accommodation portion 210. At this time, the accommodation portion 210 moves in the second direction, but one side of the injection hole 220 may be fixed to the reservoir 103. That is, the position of the injection hole 220 in the housing 101 does not change, but the position of the other side of the injection hole 220 in the accommodation portion 210 may change due to the rising of the accommodation portion 210 in the second direction.

For example, when the other side of the injection hole 220 is pressed deeply into the accommodation portion 210, the injection hole 220 may be opened. Accordingly, the inhalable composition in the accommodation portion 210 may move to the reservoir 103 through the injection hole 220. At this time, the inhalable composition may move by a pressure difference between the reservoir 103 and the accommodation portion 210. That is, when the inhalable composition filled in the reservoir 103 by the user is consumed as the aerosol is discharged, the pressure in the reservoir 103 may be reduced, and therefore, when the injection hole 220 is opened, the inhalable composition may move from the accommodation portion 210 to the reservoir 103. On the other hand, when the reservoir 103 is sufficiently filled with the inhalable composition, the inhalable composition may not additionally move to the reservoir 103 even if the user presses the lever 300 to open the injection hole 220.

Hereinafter, referring to FIGS. 4 and 5, a process in which the lever 300 of the inhaler 10 according to an embodiment operates in two steps to sequentially open the relief valve 700 and the canister 200 and return to its original position will be described.

FIG. 4A shows the inhaler 10 in a state where no external force is applied to the lever 300.

At this time, as shown in FIG. 5A, both the relief valve 700 and the canister 200 are not opened.

FIG. 4B shows the inhaler 10 in a state where the lever 300 is pressed in the first direction and moved to the first position.

At this time, the support member 310 restricts the rotation of the end portion of the protrusion member 320 in a direction away from the canister 200. Accordingly, the protrusion member 320 may come into contact with the other end of the relief bar 800 in a state where the end portion stands up toward the first surface of the housing 101. The relief bar 800 raises in the second direction by the protrusion member 320 to open the relief valve 700. As shown in FIG. 5B, as the relief valve 700 is opened, the composition remaining in the reservoir 103 is discharged to the outside.

FIG. 4C shows the inhaler 10 in a state where the lever 300 is further pressed in the first direction in the state of FIG. 4B and moved to the second position.

As the lever 300 passes the first position, the protrusion member 320 may escape from the contact with the end of the relief bar 800 and the relief bar 800 may return to its original position. That is, the relief bar 800 moves down and the relief valve 700 is closed. In addition, as shown in FIG. 5C, when the lever 300 eventually reaches the second position, the inclined surface of the wedge member 330 raises the accommodation portion 210 of the canister 200 in the second direction, thereby opening the injection hole 220. The inhalable composition may move through the injection hole 220 and the inhalable composition may be smoothly filled in the reservoir 103 from which the residual gas is removed.

FIG. 4D shows the inhaler 10 in a state where the lever 300 has reached the first position while returning to the original position from the second position.

At this time, the inclined surface 3301 moves in a direction opposite to the first direction, and the accommodation portion 210 moves down and the injection hole 220 is closed as shown in FIG. 5D. In other words, the filling of the reservoir 103 with the inhalable composition is complete. The protrusion member 320 may come into contact with the other end of the relief bar 800 at the same time as the contact between the inclined surface 3301 and the bottom surface of the accommodation portion 210 is released. At this time, the support member 310 allows the end portion of the protrusion member 320 to rotate toward the canister 200. Accordingly, the end portion of the protrusion member 320 may not maintain the standing state, but may smoothly pass the other end of the relief bar 800 while rotating toward the canister 200. For example, the protrusion member 320 may avoid the relief bar 800 elastically. As a result, when it moves in a direction opposite to the first direction, the protrusion member 320 does not raise the relief bar 800 in the second direction. Accordingly, the relief valve 700 may remain closed, and the inhalable composition newly filled in the reservoir 103 is not discharged to the outside.

FIG. 4E shows the inhaler 10 in a state where the lever 300 is completely returned to the original position.

As shown in FIG. 5E, the protrusion member 320 which has passed the relief bar 800 may return to the same position as the protrusion member 320 of FIG. 5A. For example, the protrusion member 320 may be provided with an elastic member (not shown) that is compressible in a direction in which the end portion of the protrusion member 320 rotates toward the canister 200. In the operation of 5D, the elastic member may be compressed in a direction in which the end portion of the protrusion member 320 rotates toward the canister 200, and if no external force is applied to the protrusion member 320, the elastic member may rotate the protrusion member 320 to its original position as shown in FIGS. 5A and 5E. Accordingly, when the lever 300 returns to the original position after the filling is finished, the protrusion member 320 may elastically escape the relief bar 800 without pushing it up to return to the original position.

In the inhaler 10 according to an embodiment described above, in order to prevent pressure of a residual gas remaining in the reservoir 103 from hindering a new composition from entering when the reservoir 103 is refilled with the composition using the filling lever 300, the relief valve 700 interlocked with the filling lever 300 may be provided to remove the residual gas in the reservoir 103 before the filling.

In addition, in the inhaler 10 according to an embodiment described above, the filling lever 300 may operate in two steps. In a first step, the relief valve 700 may be opened as a state before the filling, and in a second step, the relief valve 700 may be closed, thereby performing the filling.

## Claims

1. An inhaler (10) comprising:
a housing (101) having one surface, the other surface opposite to the one surface, and a plurality of side surfaces connecting the one surface and the other surface;
a reservoir (103) which is disposed inside the housing (101) and is configured to store an inhalable composition;
a canister (200) which is configured to accommodate the inhalable composition to be stored in the reservoir (103) and is connected to the reservoir (103) to be opened or closed;
a relief valve (700) which is disposed to be spaced apart from the canister (200) and provided to open and close the reservoir (103) to the outside; and
a lever (300) which is configured to control an opening and closing operation of the canister (200) and the relief valve (700),
wherein the lever (300) is configured to control the canister (200) to be opened after the relief valve (700) is opened.

2. The inhaler of claim 1, further comprising:
a relief bar (800) having one end coupled to the relief valve (700) and extending toward the lever (300),
wherein the relief bar (800) is positioned between the lever (300) and the canister (200).

3. The inhaler of claim 2,
wherein the lever (300) further comprises:
a support member (310) which extends in a first direction toward the canister (200); and
a protrusion member (320) having an end portion rotatably coupled to the support member (310) around a rotation shaft provided in the support member (310), and
wherein the other end of the relief bar (800) is positioned between the protrusion member (320) and the other surface of the housing (101).

4. The inhaler of claim 3, wherein, when the lever (300) is pressed, the protrusion member (320) moves the relief bar (800) in a second direction toward the one surface of the housing (101) while being moved in the first direction.

5. The inhaler of claim 3,
wherein, when the lever (300) is pressed in the first direction, the support member (310) restricts rotation of the protrusion member (320), and
wherein, when the lever (300) returns to an original position, the support member (310) allows the rotation of the protrusion member (320).

6. The inhaler of claim 3, wherein the protrusion member (320) is disposed so that rotation in a direction toward the canister (200) is not restrained and rotation in a direction away from the canister (200) is restrained in a state where the end portion stands toward the one surface of the housing (101).

7. The inhaler of claim 3, wherein, when the canister (200) is opened, the protrusion member (320) is positioned between the relief bar (800) and the canister (200).

8. The inhaler of claim 3,
wherein the lever (300) further comprises a wedge member (330) extending toward a bottom surface of the canister (200), and
wherein a distance between the wedge member (330) and the bottom surface of the canister (200) is greater than a distance between the protrusion member (320) and the relief bar (800).

9. The inhaler of claim 8,
wherein the wedge member (330) comprises an inclined surface (3301) inclined to be adjacent to the bottom surface along the first direction, and
wherein, when the lever (300) is further pressed in the first direction in a state where the inclined surface (3301) comes into contact with the bottom surface, the inclined surface (3301) moves the canister (200) in the second direction toward the one surface of the housing (101).

10. The inhaler of claim 1,
wherein, when the lever (300) reaches a first position in which the relief valve (700) is opened, the inhalable composition remaining in the reservoir (103) is discharged to the outside, and
wherein, when the lever (300) reaches a second position in which one side of the canister (200) is opened, the reservoir (103) is filled with the inhalable composition accommodated in the canister (200).

11. The inhaler of claim 2, wherein the relief bar (800) opens the relief valve (700) when the relief bar (800) moves in a second direction toward the one surface of the housing (101), and closes the relief valve (700) when the relief bar (800) returns to an original position.

12. The inhaler of claim 1,
wherein the canister (200) comprises:
an accommodation portion (210) which is configured to accommodate the inhalable composition; and
an injection hole (220) having one side connected to the reservoir (103) and the other side positioned in the accommodation portion (210), and
wherein the injection hole (220) is opened or closed to allow or restrict movement of the inhalable composition to the reservoir (103).

13. The inhaler of claim 12, wherein the injection hole (220) is opened when the accommodation portion (210) moves in a second direction from the other surface to the one surface of the housing (103), and is closed when the accommodation potion (210) returns to an original position.

## Patentansprüche

1. Inhalator (10), der Folgendes umfasst:
ein Gehäuse (101), das eine Oberfläche, eine andere Oberfläche gegenüber der einen Oberfläche und mehrere Seitenflächen, die die eine Oberfläche und die andere Oberfläche verbinden, aufweist;
ein Reservoir (103), das im Gehäuse (101) angeordnet ist und konfiguriert ist, eine inhalierbare Zusammensetzung zu speichern;
ein Gefäß (200), das konfiguriert ist, die inhalierbare Zusammensetzung aufzunehmen, die im Reservoir (103) gespeichert werden soll, und das so mit dem Reservoir (103) verbunden ist, dass es geöffnet oder geschlossen wird;
ein Entlastungsventil (700), das so angeordnet ist, dass es vom Gefäß (200) beabstandet ist und dazu vorgesehen ist, das Reservoir (103) zur Außenseite hin zu öffnen und zu schließen; und
einen Hebel (300), der konfiguriert ist, einen Öffnungs- und Schließvorgang des Gefäßes (200) und des Entlastungsventils (700) zu steuern,
wobei der Hebel (300) konfiguriert ist, das Gefäß (200) so zu steuern, dass es geöffnet wird, nachdem das Entlastungsventil (700) geöffnet worden ist.

2. Inhalator nach Anspruch 1, der ferner Folgendes umfasst:
eine Entlastungsstange (800), wovon ein Ende mit dem Entlastungsventil (700) gekoppelt ist und die sich zum Hebel (300) hin erstreckt,
wobei die Entlastungsstange (800) zwischen dem Hebel (300) und dem Gefäß (200) positioniert ist.

3. Inhalator nach Anspruch 2,
wobei der Hebel (300) ferner Folgendes umfasst:
ein tragendes Element (310), das sich in einer ersten Richtung zum Gefäß (200) erstreckt; und
ein vorstehendes Element (320), wovon ein Endabschnitt mit dem tragenden Element (310) um eine drehbare Welle, die im tragenden Element (310) vorgesehen ist, drehbar gekoppelt ist, und
wobei das andere Ende der Entlastungsstange (800) zwischen dem vorstehenden Element (320) und der anderen Oberfläche des Gehäuses (101) positioniert ist.

4. Inhalator nach Anspruch 3, wobei dann, wenn der Hebel (300) gedrückt wird, das vorstehende Element (320) die Entlastungsstange (800) in einer zweiten Richtung zu der einen Oberfläche des Gehäuses (101) bewegt, während es in der ersten Richtung bewegt wird.

5. Inhalator nach Anspruch 3,
wobei dann, wenn der Hebel (300) in der ersten Richtung gedrückt wird, das tragende Element (310) die Drehung des vorstehenden Elements (320) einschränkt, und
wobei dann, wenn der Hebel (300) zu einer Ursprungsposition zurückkehrt, das tragende Element (310) die Drehung des vorstehenden Elements (320) zulässt.

6. Inhalator nach Anspruch 3, wobei das vorstehende Element (320) so angeordnet ist, dass eine Drehung in einer Richtung zum Gefäß (200) hin nicht eingeschränkt ist, und eine Drehung in einer Richtung weg vom Gefäß (200) in einem Zustand eingeschränkt ist, in dem der Endabschnitt in Richtung der einen Oberfläche des Gehäuses (101) zeigt.

7. Inhalator nach Anspruch 3, wobei dann, wenn das Gefäß (200) geöffnet ist, das vorstehende Element (320) zwischen der Entlastungsstange (800) und dem Gefäß (200) positioniert ist.

8. Inhalator nach Anspruch 3,
wobei der Hebel (300) ferner ein Keilelement (330) umfasst, das sich zu einer Bodenfläche des Gefäßes (200) erstreckt, und
wobei ein Abstand zwischen dem Keilelement (330) und der Bodenfläche des Gefäßes (200) größer als ein Abstand zwischen dem vorstehenden Element (320) und der Entlastungsstange (800) ist.

9. Inhalator nach Anspruch 8,
wobei das Keilelement (330) eine schräg verlaufende Oberfläche (3301) umfasst, die so geneigt ist, dass sie längs der ersten Richtung an die Bodenfläche angrenzt, und
wobei dann, wenn der Hebel (300) in einem Zustand weiter in der ersten Richtung gedrückt wird, in dem die schräg verlaufende Oberfläche (3301) mit der Bodenfläche in Kontakt kommt, die schräg verlaufende Oberfläche (3301) das Gefäß (200) in der zweiten Richtung zu der einen Oberfläche des Gehäuses (101) hin bewegt.

10. Inhalator nach Anspruch 1,
wobei dann, wenn der Hebel (300) eine erste Position erreicht, bei der das Entlastungsventil (700) geöffnet ist, die inhalierbare Zusammensetzung, die im Reservoir (103) verbleibt, nach außen abgeführt wird, und
wobei dann, wenn der Hebel (300) eine zweite Position erreicht, bei der eine Seite des Gefäßes (200) geöffnet ist, das Reservoir (103) mit der inhalierbaren Zusammensetzung gefüllt wird, die im Gefäß (200) aufgenommen ist.

11. Inhalator nach Anspruch 2, wobei die Entlastungsstange (800) das Entlastungsventil (700) öffnet, wenn sich die Entlastungsstange (800) in einer zweiten Richtung zu der einen Oberfläche des Gehäuses (101) hin bewegt, und das Entlastungsventil (700) schließt, wenn die Entlastungsstange (800) zu einer Ursprungsposition zurückkehrt.

12. Inhalator nach Anspruch 1,
wobei das Gefäß (200) Folgendes umfasst:
einen Aufnahmeabschnitt (210), der konfiguriert ist, die inhalierbare Zusammensetzung aufzunehmen; und
ein Einspritzloch (220), wovon eine Seite mit dem Reservoir (103) verbunden ist und die andere Seite im Aufnahmeabschnitt (210) positioniert ist, und
wobei das Einspritzloch (220) geöffnet oder geschlossen wird, um eine Bewegung der inhalierbaren Zusammensetzung zum Reservoir (103) zu ermöglichen oder einzuschränken.

13. Inhalator nach Anspruch 12, wobei das Einspritzloch (220) geöffnet ist, wenn sich der Aufnahmeabschnitt (210) in einer zweiten Richtung von der anderen Oberfläche zu der einen Oberfläche des Gehäuses (103) bewegt, und geschlossen ist, wenn der Aufnahmeabschnitt (210) zu einer Ursprungsposition zurückkehrt.

## Revendications

1. Inhalateur (10) comportant :
un boîtier (101) ayant une première surface, la seconde surface étant opposée à la première surface, et une pluralité de surfaces latérales reliant la première surface et la seconde surface ;
un réservoir (103) qui est disposé à l'intérieur du boîtier (101) et est configuré pour stocker une composition inhalable ;
une cartouche (200) qui est configurée pour recevoir la composition inhalable à stocker dans le réservoir (103) et est reliée au réservoir (103) pour être ouverte ou fermée ;
une soupape de décharge (700) qui est disposée de manière à être espacée de la cartouche (200) et agencée de manière à ouvrir et fermer le réservoir (103) vers l'extérieur ; et
un levier (300) qui est configuré pour commander une opération d'ouverture et de fermeture de la cartouche (200) et de la soupape de décharge (700),
dans lequel le levier (300) est configuré pour commander l'ouverture de la cartouche (200) après l'ouverture de la soupape de décharge (700).

2. Inhalateur selon la revendication 1, comportant en outre :
une barrette de décharge (800) ayant une extrémité couplée à la soupape de décharge (700) et s'étendant vers le levier (300),
dans lequel la barrette de décharge (800) est positionnée entre le levier (300) et la cartouche (200).

3. Inhalateur selon la revendication 2,
dans lequel le levier (300) comporte en outre :
un élément de support (310) qui s'étend dans une première direction allant vers la cartouche (200) ; et
un élément de saillie (320) ayant une partie d'extrémité couplée en rotation à l'élément de support (310) autour d'un axe de rotation agencé dans l'élément de support (310), et
dans lequel la seconde extrémité de la barrette de décharge (800) est positionnée entre l'élément de saillie (320) et la seconde surface du boîtier (101).

4. Inhalateur selon la revendication 3, dans lequel, lorsque le levier (300) est pressé, l'élément de saillie (320) déplace la barrette de décharge (800) dans une seconde direction allant vers la surface du boîtier (101) tout en étant déplacé dans la première direction.

5. Inhalateur selon la revendication 3,
dans lequel, lorsque le levier (300) est pressé dans la première direction, l'élément de support (310) restreint une rotation de l'élément de saillie (320), et
dans lequel, lorsque le levier (300) revient à une position d'origine, l'élément de support (310) permet la rotation de l'élément de saillie (320).

6. Inhalateur selon la revendication 3, dans lequel l'élément de saillie (320) est disposé de sorte qu'une rotation dans un sens allant vers la cartouche (200) n'est pas restreinte et une rotation dans un sens s'éloignant de la cartouche (200) est restreinte dans un état où la partie d'extrémité se situe vers la première surface du boîtier (101).

7. Inhalateur selon la revendication 3, dans lequel, lorsque la cartouche (200) est ouverte, l'élément de saillie (320) est positionné entre la barrette de décharge (800) et la cartouche (200).

8. Inhalateur selon la revendication 3,
dans lequel le levier (300) comporte en outre un élément de coin (330) s'étendant vers une surface inférieure de la cartouche (200), et
dans lequel la distance entre l'élément de coin (330) et la surface inférieure de la cartouche (200) est supérieure à la distance entre l'élément de saillie (320) et la barre de décharge (800).

9. Inhalateur selon la revendication 8,
dans lequel l'élément de coin (330) comporte une surface inclinée (3301) inclinée de manière à être adjacente à la surface inférieure le long de la première direction, et
dans lequel, lorsque le levier (300) est pressé plus loin dans la première direction dans un état où la surface inclinée (3301) vient en contact avec la surface inférieure, la surface inclinée (3301) déplace la cartouche (200) dans la seconde direction allant vers la première surface du boîtier (101).

10. Inhalateur selon la revendication 1,
dans lequel, lorsque le levier (300) atteint une première position dans laquelle la soupape de décharge (700) est ouverte, la composition inhalable restant dans le réservoir (103) est évacuée vers l'extérieur, et
dans lequel, lorsque le levier (300) atteint une seconde position dans laquelle un côté de la cartouche (200) est ouvert, le réservoir (103) est rempli de la composition inhalable reçue dans la cartouche (200).

11. Inhalateur selon la revendication 2, dans lequel la barrette de décharge (800) ouvre la soupape de décharge (700) lorsque la barrette de décharge (800) se déplace dans une seconde direction allant vers la première surface du boîtier (101), et ferme la soupape de décharge (700) lorsque la barrette de décharge (800) revient à une position d'origine.

12. Inhalateur selon la revendication 1,
dans lequel la cartouche (200) comporte :
une partie de réception (210) qui est configurée pour recevoir la composition inhalable ; et
un trou d'injection (220) ayant un côté relié au réservoir (103) et l'autre côté positionné dans la partie de réception (210), et
dans lequel le trou d'injection (220) est ouvert ou fermé pour permettre ou restreindre un mouvement de la composition inhalable jusqu'au réservoir (103).

13. Inhalateur selon la revendication 12, dans lequel le trou d'injection (220) est ouvert lorsque la partie de réception (210) se déplace dans une seconde direction allant de la seconde surface à la première surface du boîtier (103), et est fermé lorsque la partie de réception (210) revient à une position d'origine.
